# EUROPÄISCHE PATENTSCHRIFT

(11) **EP 0 782 865 B1**
(45) Veröffentlichungstag und Bekanntmachung des Hinweises auf die Patenterteilung: **29.08.2001**
(21) Anmeldenummer: 96120714.9
(22) Anmeldetag: 21.12.1996
(51) Int. Cl.: A61L 31/00

(54) **Verwendung von Lactidpolymeren zur Adhäsionsprophylaxe**
Use of lactide polymers for prevention of adhesion
Utilisation de polymères de lactide pour la prévention d'adhésion

(30) Priorität: 03.01.1996 DE 19600095
(43) Veröffentlichungstag der Anmeldung: 09.07.1997
(73) Patentinhaber: Jürgens, Christian, Dr. med., 20149 Hamburg (DE)
(72) Erfinder: Jürgens, Christian, Dr., 20149 Hamburg (DE); Ryfger-Kricheldorf, Hans, Dr., 22607 Hamburg (DE); Kreiser-Saunders, Dr., 28195 Bremen (DE)
(74) Vertreter: Benz, Jürgen

(56) Entgegenhaltungen:
- EP-A- 0 608 139
- WO-A-92/10218
- DE-A- 4 112 489

## Beschreibung

Die Erfindung betrifft die Verwendung physiologisch unbedenklicher Folien aus Copolymeren für die Verhinderung postoperativer Adhäsionen nach chirurgischen Eingriffen.

Postoperative Adhäsion ist ein pathologisches Phänomen. Es wird durch operative oder durch unfallverursachte Traumatisierung von Gewebe bedingt, und ist eine nach Verklebung durch Fibrin entstehende bindegewebige Verwachsung. Postoperative Adhäsionen sind in der Klinik häufig anzutreffende Komplikationen, die nach fast jedem chirurgischen Eingriff auftreten können. 93 % der Patienten zeigen nach abdominaler Voroperation Adhäsionen (1) und auch der größte Teil der Dünndarmobstruktionen ist durch postoperative Adhäsionen bedingt (2, 3). Die Einflußmöglichkeiten des Operateurs sind bisher sehr gering. So ist beim sog. Verwachsungsbauch die Entstehung von Briden (Bindegewebsstränge) und Adhäsionen operationstechnisch und medikamentös kaum beeinflußbar . (3). "Jeder abdominale Eingriff birgt die Gefahr eines späteren Verwachsungsbauches in sich." (3).

Der bisherige Stand der Technik läßt sich wie folgt zusammenfassen:

Die primäre Methode der Adhäsionsprophylaxe ist die möglichst saubere Präparation des traumatisierten Gewebes. Einen weiteren Ansatzpunkt bietet das Fibrin, da die vorangehende Verklebung mit Fibrin die Voraussetzung für die Bildung von bindegewebigen Adhäsionen und Briden ist. Daher wird versucht, durch Spülung mit physiologischer Kochsalzlösung insbesondere der Bauchhöhle nach langwierigen abdominalen Eingriffen sowie durch den örtlichen Einsatz von Fibrinolytika eine Verklebung von Fibrin zu verhindern oder aufzulösen. Der Erfolg dieser Methoden ist jedoch gering, da stets nur ein Teil der Verklebungen betroffen ist.

Darüberhinaus werden in der Chirurgie verschiedentlich Vliese, Gitter und Folien aus resorbierbaren Copolymeren aus Glykolid und Läctid eingesetzt. Diese sollen Heilungsprozeße erleichtern oder ermöglichen, so z.B. bei einer Milzläsion, oder Organe nach einer Amputation in ihrer unmittelbaren Umgebung in Position halten, wie z.B. bei Abschluß des kleinen Beckens gegenüber der Bauchhöhle nach einer Rektumamputation (4). Sie werden aber nicht zur Verhinderung von Adhäsionen eingesetzt (4), sondern gelegentlich sogar zur gezielten Narbenbildung z.B. bei Leistenbruch.

Unter den in der Medizin angewandten Kunststoffen nehmen hochpolymere Hydroxycarbonsäuren wie Polylactid oder Polyglykolid einen besonderen Platz ein. Sie werden seit längerem in der Chirurgie als resorbierbare Fäden oder Osteosyntheseimplantate sowie als Folien zur Wundabdeckung eingesetzt. Ein erfindungsgemäß verwendbares Material, das in den Patentschrift DE - P 41 12 489 beschrieben wurde, zeichnet sich durch höhere Flexibilität und Haftung gegenüber anderen wundabdeckenden Materialien wie den in den Patenten DE - OS 36 20 685 oder FR - OS 2126270 beschriebenen aus. Das gilt auch gegenüber den aus dem Patent WO 84/04331 bekannten thermoplastischen Copolymeren, die als Filme oder Überzüge für Carbonfasersubstrate verwendet werden, die als Körperimplantate zur Ausheilung geschädigter Muskelbänder oder Sehnen bzw. Ersatzimplantate zur Verwendung kommen. Aber zur Adhäsionsprophylaxe wurden diese Materialien bisher ebenfalls nicht eingesetzt.

EP-0 608 139 beschreibt bioresorbierbare Elastomere, die als Filme zur Adhäsionsprophylaxe geeignet sind. Sie bestehen aus 30-50% ε-Caprolacton, trimethylencarbonat, Etherlacten oder einer Mischung dieser Komponenten und Glycolid und/oder Paradioxanon. Die Resorptionszeit für diese Elastomere liegt bei 6 Monaten.

Es besteht daher ein Bedarf nach einem Mittel zur Verhinderung postoperativer Adhäsionen.

Erfindungsgemäß wird nun die Verwendung resorbierbarer physiologisch unbedenklicher Folien aus Copolymeren für die Verhinderung postoperativer Adhäsionen und Briden nach chirurgischen Eingriffen vorgeschlagen, wobei die Copolymere durch Umsetzung der Monomere im Molverhältnis Lactid zu Reaktionspartner von 90 bis 70 zu 30 bis 10 unter Zugabe von Zinn(II)-diethylhexanoat als Initiator, einem Verhältnis von Reaktionspartner zu Initiator von 300 : 1 bis über 1000 : 1 bei Temperaturen von 130 - 150°C über eine Zeitspanne von 24 bis 96 Stunden hergestellt werden. Die so beschriebenen Copolymere sind für die topische Anwendung auf menschlicher oder tierischer Haut geeignet und denen ähnlich, die bereits für topische Zwecke benutzt werden, z.B. als Incisionsfolie für Operationen, als Sonnenschutz oder Insektenabwehrmittel nach Zusatz geeigneter Wirkstoffe, als Spray oder Folie zur Wundabdeckung oder als flüssiger Handschuh. Weitere Untersuchungen sowie praktische Anwendungen haben überraschend gezeigt, daß diese Copolymere zur Verhinderung von postoperativen Adhäsionen an traumatisiertem Gewebe verwendet werden können. Es war bisher angenommen worden, daß sich Adhäsionen durch Folien hindurch bilden würden, wobei Poren in der Struktur ausgenutzt würden oder daß die Ausbildung von Adhäsionen durch Polymere sogar induziert werden könnte. Um so erfreulicher war es, festzustellen, daß die Folien eine wirkungsvolle Barriere gegen Fibrinverklebungen zwischen dem traumatisierten Gewebe als solchen und ggf. dem umgebenden Bindegewebe bilden und aufgrund ihrer physiologischen Unbedenklichkeit, mangelnden Toxizität und Resorbierbarkeit dafür hervorragend geeignet sind.

Die erfindungsgemäß verwendeten Copolymere sind der Kettenlänge nach als Poly- und Oligomere anzusehen und liegen als Gemisch aus Mono-, Oligo- und Polymeren vor. Es handelt sich um farblose transparente Verbindungen, die je nach dem Molverhältnis der Monomeranteile zäh verfließlich bis starr sind. Hergestellt werden die beanspruchten Verbindungen durch Umsetzung der Monomere im Molverhältnis von Lactid zu Reaktionspartner von 90 bis 70 : 10 bis30, wobei die Erhöhung des Lactidanteiles zu einer Erhöhung der Erweichungstemperatur führt. Die Umsetzung erfolgt unter Zugabe von Zinn (II)-diethylhexanoat als Initiator bei Temperaturen um 130 - 150°C während einer Zeitdauer von 24 bis 96 Stunden. Das Verhältnis von Reaktionsmischung zu Initiator beträgt 300 : 1 bis 500 : 1, wobei bei Erhöhung dieses Verhältnisses bei der Reaktion der Anteil der längeren Molekülketten erhöht wird und dadurch auch eine Erhöhung des Erweichungspunktes bedingt ist. Als Initiator wird Zinn-II-diethylhexanoat eingesetzt, da sich herausgestellt hat, daß andere an und für sich bekannte Initiatoren in der Regel eine geringere Ausbeute ergeben oder zu Trübung/Färbung führen. Außerdem sollte eine Temperatur von 130 - 150°C und eine Umsetzungszeit von 24 bis 96 Stunden nicht überschritten werden. Die Reaktion wird nach der gewünschten Umsetzungsdauer entweder durch Abkühlung der Umsetzungsmischung oder durch Zugabe von beispielsweise Chelatisierungsmitteln abgebrochen. Zur Entfernung von verbleibenden Monomeren, kurzkettigen Oligomeren oder ggf. auch überschüssigen Weichmachern kann die Reaktionsmasse in der Regel mit der 600- bis 800-fachen Menge Alkohol ausgefällt werden; ggf. kann die Reaktionsmasse auch mit Wasser oder einer wäßrigen Lösung ausgewaschen werden, da sich hierdurch auch bereits ein großer Teil von Monomeren, kurzkettigen Oligomeren oder Weichmachern entfernen läßt.

Falls erwünscht, können der Reaktionsmasse Weichmacher zugesetzt werden, um die Erweichungstemperatur und die Diffundibilität zu verändern. Als Weichmacher werden vorzugsweise überschüssiges Caprolacton, Tributylcitrat, Phthalsäureester oder Glycerin verwendet. Der Anteil an Weichmachern sollte in der Regel 10 bis 20% bezogen auf das Gewicht, nicht überschreiten. Glycerin hat als Weichmacher besondere Vorzüge, da es über eine gewisse Bakterizidie verfügt und die Diffundibilität der Folien erhöht.

Die erfindungsgemäßen Copolymere können in geeigneten organischen Lösungsmitteln wie beispielsweise Essigester, Aceton, Methylenchlorid oder THF in Lösung gebracht werden. Sie lassen sich ohne weiteren Zusatz zu Folien auswalzen, die allerdings beispielsweise auch durch Verdunsten hergestellt werden können. Andere für Kunststoffe geeignete Verarbeitungsmethoden sind ebenfalls anwendbar.

Sowohl die Oligo- als auch die Polymere werden *in vivo* und *in vitro* durch Hydrolyse abgebaut. Die Copolymere und ihre Abbauprodukte sind medizinisch unbedenklich und nicht allergen. Die Monomere werden *in vivo* über den Milchsäurezyklus bzw. über den Fettsäurestoffwechsel weiter metabolisiert. Es hat sich herausgestellt, daß die Zeiten für den hydrolytischen Abbau um so kürzer sind, je höher der Lactidanteil liegt.

Die erfindungsgemäß beanspruchte Verwendung von Folien aus den oben beschriebenen Polymeren ist die Abdeckung traumatisierten Gewebes nach einem chirurgischen Eingriff zur Verhinderung von Adhäsionen, wobei der Folie auch Pharmaka, insbesondere Antibiotika zugesetzt sein können. Durch Abdeckung der Wunde wird mechanisch eine unerwünschte Verklebung mit Fibrin zwischen dieser und anderen Bindegeweben während des Heilungsprozesses verhindert. Damit werden gleichzeitig Adhäsions- und Bridenbildung wirksam ausgeschlossen. Der große Vorteil der erfindungsgemäß verwendeten Materialien liegt darin, daß sie zu unbedenklichen Produkten abgebaut werden können und somit keinen weiteren chirurgischen Eingriff zu ihrer Entfernung notwendig machen, einfach applizierbar sind und einer geringen Ablösbarkeit durch wäßrige Flüssigkeiten wie Blut oder Lymphe unterliegen. Außerdem bietet der natürliche hydrolytische Abbau der Folie mit etwa 3 bis 30 Wochen *in vitro* dem traumatisiertem Gewebe genug Zeit zur Heilung, ohne daß es zu Adhäsionsbildungen kommen kann. Die beschriebenen Folien eignen sich durch ihre hohe mechanische Belastbarkeit und gute Anheftung besonders für Gewebe, die mechanischen Reizungen ausgesetzt sind, wie Muskeln aber auch Darmschlingen.

Die Erfindung wird im folgenden anhand der Beispiele näher erläutert:

### Beispiel 1

### Herstellung einer Folie aus den Copolymeren

Zur Herstellung einer Folie aus den Copolymeren werden D L-Lactid und ε-Caprolacton in einem Molverhältnis von 85 : 15, entsprechend 350,5 Gramm zu 49,0 Gramm langsam auf 150°C erhitzt. Sodann wird als Polymerisationsinitiator 1,16 ml Zinn-II-diethyl-hexanoat (Verhältnis Reaktionsmasse : Initiator = 500 : 1) zugegeben. Die Polymerisation erfolgt über 24 Stunden bei 150°C im Ölbad. Anschließend wird die Mischung abgekühlt und bei 70°C mit Ethylacetat auf 5 Liter aufgefüllt. Diese Lösung verbleibt dann für 36 Stunden auf einer Schüttelmaschine und kann danach als solche zur Herstellung von Folien verwendet werden.

In entsprechender Weise und Berücksichtigung der Molverhältnisse lassen sich auch die Copolymere von Lactid, evt. unter Zusatz von anderen Lactonen wie Valerolacton, Heptalacton, Decalacton oder auch von β-Hydroxybuttersäuren herstellen.

### Beispiel 2

### Verwendung einer Folie zur Abdeckung von traumatisiertem Gewebe nach einem invasiven chirurgischen Eingriff.

Die nach Beispiel 1 hergestellte Folie wird vor dem Ende eines operativen Eingriffes so auf die traumatisierten Gewebe aufgetragen, daß diese ganz bedeckt werden. Anschließend wird der chirurgische Eingriff durch Verschließen der Incision abgeschlossen.

### Literatur:

(1) Menzies D. und Ellis H., Ann. Rev. R. Coll. Surg. Eng., 1990, 72 : 60.
(2) Menzies D., Surg. Ann., 1992, 24 : 27 - 45.
(3) Schumpelick V., Bleese N.M. und Mommsen U. (Hrsg.), Chirurgie, 3. Aufl., 1994, Ferd. Enke Verlag, Stuttgart.
(4) Röntgen L. und Ulrich B., Neue Hilfsmittel und Techniken in der Allgemeinchirurgie, in der Reihe: Praktische Chirurgie, Bd. 104, 1994, Ferd. Enke Verlag, Stuttgart.

## Patentansprüche

1. Verwendung resorbierbarer physiologisch unbedenklicher Folien aus Copolymeren zur Herstellung eines Medizinproduktes zur Verhinderung postoperativer Adhäsionen nach chirurgischen Eingriffen, **dadurch gekennzeichnet**, dass die Folien aus racemischen Lactid und ε-Caprolacton, δ-Valerolacton, γ-Decalacton oder β-Hydroxybuttersäure erhältlich durch die Umsetzung der Monomere im Molverhältnis Lactid zu Reaktionspartner von 90 bis 70 : 30 bis 10 unter Zugabe von Zinn(II)-diethylhexanoat als Initiator, einem Verhältnis von Reaktionspartner zu Initiator von 300 : 1 bis über 1000 : 1 bei Temperaturen von 130 - 150 °C über eine Zeitspanne von 24 bis 96 Stunden, bestehen.

2. Verwendung nach Anspruch 1 **dadurch gekennzeichnet**, dass die Folien zusätzlich arzneilich wirksame Substanzen, insbesondere Antibiotika enthalten.

3. Verwendung nach Anspruch 1 bis 2, **dadurch gekennzeichnet**, dass die Folien physiologisch unbedenkliche Weichmacher, insbesondere Glycerin, enthalten.

## Claims

1. Use of absorbable physiologically acceptable sheets of copolymers for producing a medical device for preventing postoperative adhesions after surgical procedures, **characterized in that** the sheets consist of racemic lactide and ε-caprolactone, δ-valerolactone, γ-decalactone or β-hydroxybutyric acid, obtainable by reacting the monomers in the molar ratio of lactide to partner in the reaction of from 90 to 70 : 30 to 10 with the addition of tin(II) diethylhexanoate as initiator, with a ratio of partner in the reaction to initiator of from 300 : 1 to more than 1000 : 1 at temperatures of 130 - 150°C over a period of 24 to 96 hours.

2. Use according to Claim 1, **characterized in that** the sheets additionally contain medicinally active substances, in particular antibiotics.

3. Use according to Claims 1 to 2, **characterized in that** the sheets contain physiologically acceptable plasticizers, in particular glycerol.

## Revendications

1. Utilisation de pellicules constituées de copolymères, résorbables et physiologiquement sans inconvénient, pour la fabrication d'un produit médical destiné à empêcher des adhérences post-opératoires après des interventions chirurgicales, **caractérisée en ce que** les pellicules peuvent être obtenues à partir de lactide racémique et d'ε-caprolactone, de δ-valérolactone, de γ-décalactone ou d'acide β-hydroxybutyrique, par la réaction des monomères en un rapport molaire lactide à partenaire réactionnel de 90 à 70 : 30 à 10, avec addition de diéthylhexanoate stanneux en tant qu'amorceur, avec un rapport des partenaires réactionnels à l'amorceur de 300 : 1 à plus de 1 000 : 1 à des températures de 130-150°C, pendant une durée de 24 à 96 heures.

2. Utilisation selon la revendication 1, **caractérisée en ce que** les pellicules contiennent des substances à activité médicamenteuse, en particulier des antibiotiques.

3. Utilisation selon les revendications 1 et 2, **caractérisé en ce que** les pellicules contiennent des plastifiants physiologiquement sans inconvénient, en particulier du glycérol.
